# EUROPÄISCHE PATENTSCHRIFT

(11) **EP 1 337 466 B1**
(45) Veröffentlichungstag und Bekanntmachung des Hinweises auf die Patenterteilung: **06.05.2004**
(21) Anmeldenummer: 01993582.4
(22) Anmeldetag: 24.10.2001
(51) Int. Cl.: C01C 1/04, C01B 3/02, C01B 3/38, C01B 3/48, C01B 3/52

(54) **VERFAHREN ZUM HERSTELLEN VON AMMONIAK AUS EINEM STICKSTOFF-WASSERSTOFF-GEMISCH AUS ERDGAS**
METHOD FOR PRODUCING AMMONIA ON THE BASIS OF A NITROGEN-HYDROGEN MIXTURE FROM NATURAL GAS
PROCEDE DE PRODUCTION D'AMMONIAC A PARTIR D'UN MELANGE D'AZOTE ET D'HYDROGENE PROVENANT DE GAZ NATUREL

(30) Priorität: 10.11.2000 DE 10055818
(43) Veröffentlichungstag der Anmeldung: 27.08.2003
(73) Patentinhaber: MG Technologies AG, 60388 Frankfurt am Main (DE); Ammonia Casale S.A., 6900 Lugano (CH)
(72) Erfinder: DAVEY, William, 60439 Frankfurt am Main (DE); FILIPPI, Ermanno, 6976 Castagnola (CH)
(86) Internationale Anmeldenummer: PCT/EP2001/012254
(87) Internationale Veröffentlichungsnummer: WO 2002/038499

(56) Entgegenhaltungen:
- EP-A- 0 307 983
- EP-A- 0 905 127
- WO-A-01/09038
- FR-A- 2 368 435
- GB-A- 2 048 840

## Beschreibung

Die Erfindung betrifft ein Verfahren zum katalytischen Erzeugen von Ammoniak aus einem Stickstoff-Wasserstoff-Gemisch.

Aus dem deutschen Patent 2007441 ist die Erzeugung eines Ammoniak-Synthesegases bekannt, wobei man durch Vergasung von Kohlenwasserstoffen ein Rohgas erzeugt, das man entschwefelt, konvertiert, von CO₂ befreit und schließlich zum Entfernen restlicher Verunreinigungen einer Wäsche mit flüssigem Stickstoff unterzieht. Im EP-Patent 0307983 wird ein ähnliches Verfahren beschrieben, wobei konvertiertes Synthesegas vor der Ammoniak-Synthese einer Wäsche mit flüssigem Stickstoff unterzogen wird. Einzelheiten zur katalytischen Herstellung von Ammoniak finden sich in Ullmann's Encyclopedia of Industrial Chemistry, 5. Auflage, Band A2, Seiten 143 - 215, die Herstellung von Harnstoff ist dort im Band A27, Seiten 333 - 350 beschrieben. Ein Verfahren zum.kombinierten Erzeugen von Ammoniak und Harnstoff ist in EP-A-0905 127 beschrieben.

Der Erfindung liegt die Aufgabe zugrunde, bei der Ammoniak-Synthese möglichst kostengünstig zu arbeiten und ein Verfahren bereitzustellen, das auch gut für große Anlagen geeignet ist. Erfindungsgemäß gelingt dies dadurch, dass man Erdgas zusammen mit O₂-reichem Gas in einen autothermen Reformer leitet, wo man bei Temperaturen im Bereich von 900 - 1200 °C, einem Druck von 40 bis 100 bar und in Gegenwart eines Spaltkatalysators ein rohes Synthesegas erzeugt, welches, trocken gerechnet, einen H₂-Gehalt von 55 - 75 Vol.-%, einen CO-Gehalt von 15 - 30 Vol.-% einen CO₂-Gehalt von 5 bis 30 Vol.-% und ein Volumenverhältnis H₂ : CO von 1,6 - 4 aufweist, dass man das rohe Synthesegas aus dem autothermen Reformer abzieht, kühlt, durch eine katalytische Konvertierung zum Umwandeln von CO in H₂ leitet und ein konvertiertes Synthesegas mit einem H₂-Gehalt, trocken gerechnet, von mindestens 55 Vol.-% und einem CO-Gehalt von höchstens 8 Vol.-% abzieht, dass man das konvertierte Synthesegas einer mehrstufigen Gasreinigung zum Entfernen von CO₂, CO und CH₄ unterzieht, und dass man ein N₂-H₂-Gemisch erzeugt, welches man einer Ammoniak-Synthese zum katalytischen Erzeugen von Ammoniak zuführt.

Für das Verfahren ist es wichtig, dass man beim Erzeugen des rohen Synthesegases auf eine Anlage zum Dampfreformierten (steam reforming) verzichtet. Im autothermen Reformer kann man bei relativ hohen Drücken arbeiten, die im Bereich von 30 - 100 bar und zumeist 40 - 80 bar liegen. Stromab vom Reformer kann dieser hohe Druck ungefähr beibehalten werden, so dass man das Synthesegas vor Eintritt in die Ammoniak-Synthese nur wenig verdichten muss. Dies ist gegenüber konventionellen Arbeitsweisen mit Dampfreformierung, in welcher nur relativ niedrige Drücke zugelassen sind, erheblich kostengünstiger. Der autotherme Reformer hat gegenüber der Dampfreformierung den weiteren Vorteil, dass er ein Gas mit einem ausreichenden H₂/CO₂-Verhältnis liefert, so dass man nach der Konvertierung mit dem in der Gasreinigung anfallenden CO₂ das gesamte produzierte NH₃ zu Harnstoff umwandeln kann.

Eine vorteilhafte Weiterbildung besteht darin, dass man den in der Ammoniak-Synthese erzeugten Ammoniak mindestens teilweise durch Umsetzen mit CO₂ zu Harnstoff umwandelt. Dabei ist es vorteilhaft, wenn man in mindestens einer Gaswaschstufe CO₂ aus dem konvertierten Synthesegas entfernt und das entfernte CO₂ zum Erzeugen von Harnstoff verwendet. Eine von mehreren Möglichkeiten ist das in EP-A-0905 127 beschriebene kombinierte Verfahren. Üblicherweise reicht das in der Gaswaschstufe anfallende CO₂ völlig aus, um den CO₂-Bedarf der Harnstoff-Synthese zu erfüllen, im Gegensatz zu konventionellen Verfahren.

Die Entfernung von CO₂ aus dem konvertierten Gasgemisch kann vorteilhafterweise durch ein physikalisches Waschverfahren erfolgen, das z.B. mit Methanol bei Temperaturen von -20 bis -70 °C arbeitet. Hierbei wird nur relativ wenig Energie, einschließlich Kompressionsenergie, verbraucht. Gleichzeitig kann man hierbei in der Regeneration der Waschflüssigkeit mindestens die Hälfte des CO₂ bei Drücken z.B. im Bereich von 2 - 8 bar wiedergewinnen, so dass man in der nachfolgenden Verwendung des CO₂ zum Erzeugen von Harnstoff Kompressionsenergie spart.

Es ist zweckmäßig, wenn das dem autothermen Reformer zugeführte O₂-reiche Gas einen O₂-Gehalt von mindestens 70 Vol.-% und zumeist mindestens 90 Vol.-% aufweist. Auf diese Weise verringert man den Gehalt an Verunreinigungen im rohen Synthesegas und die Gaswaschstufe kann verkleinert werden.

Ausgestaltungsmöglichkeiten des Verfahrens werden mit Hilfe der Zeichnung erläutert.
- Fig. 1: zeigt ein Fließschema des Verfahrens,
- Fig. 2: zeigt ein Fließschema einer alternativen Verfahrensführung.

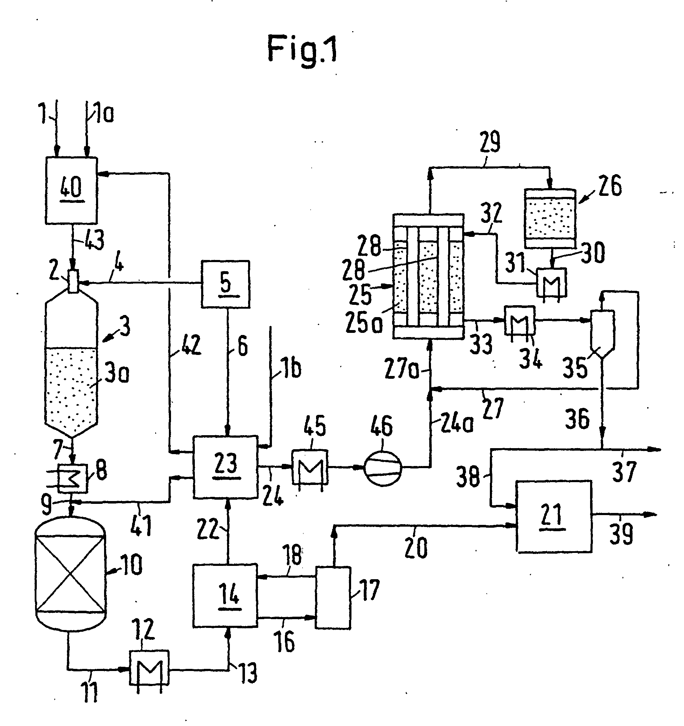

Gemäß Fig. 1 führt man der Vorbehandlung (40) Erdgas durch die Leitung (1) und Wasserdampf durch die Leitung (1a) zu, um eine Entschwefelung, Erhitzung und Entfernung der C₂₊-Komponenten in an sich bekannter Weise durchzuführen. Der Vorbehandlung (40) wird auch methanhaltiges Gas durch die Leitung (42) aufgegeben. Ein Gemisch, das vor allem aus Methan und Wasserdampf besteht, strömt in der Leitung (43) zu einem Brenner (2) eines autothermen Reformers (3) und gleichzeitig wird durch die Leitung (4) O₂-reiches Gas mit einem O₂-Gehalt von üblicherweise mindestens 70 Vol.-% und vorzugsweise mindestens 95 Vol.-% herangeführt. Das O₂-reiche Gas kommt aus einer Luftzerlegungsanlage (5). Der Reformer (3) enthält ein Festbett (3a) eines an sich bekannten körnigen Spaltkatalysators z. B. auf Nickel-Basis. Im Reaktor herrscht ein Druck im Bereich von 30 - 100 bar und vorzugsweise 40 - 80 bar, die Temperaturen liegen im Bereich von 900 bis 1200 °C. Das in der Leitung (7) abgezogene rohe Synthesegas weist einen H₂-Gehalt von 55 - 75 Vol.-%, einen CO-Gehalt von 15 - 30 Vol.-%, einen CO₂-Gehalt von 5 - 30 Vol.-% und ein Volumenverhältnis H₂ : CO von 1,6 - 4 auf. Nach Kühlung im Wärmeaustauscher (8) gibt man das rohe Synthesegas durch die Leitung (9) einer katalytischen Konvertierung (10) auf, die auch aus mehreren Reaktoren bestehen kann. Man arbeitet im Temperaturbereich von 150 - 500 °C und vorzugsweise 280 - 450 °C, wobei man an sich bekannte Katalysatoren z. B. auf Eisen-Basis verwendet. Durch die Konvertierung wandelt man CO + H₂O zu CO₂ + H₂ um. Das Gas der Leitung (11) weist vorzugsweise ein Volumenverhältnis H₂ : CO₂ von 2,5 - 3 (trocken gerechnet) auf

Das in der Leitung (11) abgezogene konvertierte Synthesegas weist einen H₂-Gehalt, trocken gerechnet, von mindestens 55 Vol.-% und vorzugsweise mindestens 65 Vol.-% und einen CO-Gehalt von höchstens 8 Vol.-% auf Dieses Gas führt man zunächst durch eine indirekte Kühlung (12) und gibt es dann durch die Leitung (13) einer Gaswaschanlage (14) auf, um insbesondere CO₂ zu entfernen. Dies kann z. B. durch eine physikalische Wäsche mit Methanol bei Temperaturen im Bereich von etwa -70 bis -20 °C erfolgen, eine andere Möglichkeit ist z. B. eine Wäsche mittels Methyldiethylamin oder die Selexol-Wäsche. Gebrauchte, CO₂-haltige Waschlösung zieht man in der Leitung (16) ab und gibt sie einer Regeneration (17) auf, um das CO₂ aus der Waschlösung zu entfernen. Regenerierte Waschlösung wird in der Leitung (18) zurück in die Gaswaschanlage (14) geführt. Das anfallende CO₂ eignet sich sehr gut dazu, um durch die Leitung (20) einer Harnstoff-Synthese (21) zugeführt zu werden.

Teilgereinigtes Synthesegas zieht aus der Gaswaschanlage (14) in der Leitung (22) ab und wird in einer zweiten Waschanlage (23) behandelt, wo flüssiger Stickstoff als Waschflüssigkeit dient. Der dazu nötige Stickstoff kommt aus der Luftzerlegungsanlage (5) und wird. in der Leitung (6) herangeführt. Einzelheiten einer Wäsche mit flüssigem Stickstoff zum Erzeugen eines NH₃-Synthesegases finden sich im EP-Patent 0307983, das bereits oben erwähnt wurde. Üblicherweise fällt in der Waschanlage (23) ein CO-haltiges Gas an, welches man durch die Leitung (41) in die Konvertierung (10) zurückführt. Wenn gleichzeitig auch ein CH₄-reiches Gas anfällt, führt man dies in der Leitung (42) zurück. Um die Kälteerzeugung zu unterstützen, führt man durch die Leitung (1b) einen Erdgas-Strom mit einem Druck von 10 bis 100 bar und vorzugsweise mindestens 30 bar heran. Diesen Strom expandiert man in der Waschanlage (23), um eine Druckemiedrigung um mindestens 8 bar und vorzugsweise mindestens 25 bar zu erreichen. Das expandierte Erdgas kann dann z.B. ebenfalls in der Leitung (42) abgeführt werden.

Die Wäsche (23) wird so geführt, dass das in der Leitung (24) anfallende Synthesegas bereits ein Molverhältnis H₂ : N₂ von etwa 3 : 1 aufweist. Dieses Synthesegas wird im indirekten Wärmeaustauscher (45) angewärmt, im Kompressor (46) komprimiert und strömt durch die Leitung (24a) zu einer Ammoniaksynthese, zu welcher der indirekt gekühlte Reaktor (25) und der adiabatisch arbeitende Reaktor (26) gehören. Im Kreislauf geführtes Synthesegas aus der Leitung (27) zusammen mit dem frischen Synthesegas der Leitung (24a) tritt durch die Leitung (27a) mit Temperaturen im Bereich von 100 - 200 °C in den Reaktor (25) ein und strömt dort durch Röhren (28) oder Kanäle, wobei das Gas als Kühlmedium dient und Wärme aus dem Katalysatorbett (25a) abführt. Alternativ kann in der Ammoniak-Synthese auch siedendes Wasser als Kühlmedium dienen.

Das Synthesegas verlässt den Reaktor (25) in der Leitung (29) mit Temperaturen im Bereich von 300 - 500 °C und kommt im Reaktor (26) mit dessen Katalysator in Kontakt, der eine Schüttung bildet. Die NH₃-bildende Reaktion ist exotherm, so dass das in der Leitung (30) abströmende Gemisch Temperaturen von 400 - 600 °C aufweist und dadurch einen Kühler (31) geführt wird. Anschließend tritt das NH₃haltige Synthesegas von der Leitung (32) kommend in den Reaktor (25) und strömt durch dessen indirekt gekühltes Katalysatorbett. Die Austrittstemperatur in der Leitung (33) liegt im Bereich von 300 - 500 °C und vorzugsweise 380 - 430 °C. Das Produktgemisch in der Leitung (33) weist eine NH₃-Konzentration von mindestens 20 Vol.-% auf, es enthält daneben vor allem noch N₂ und H₂. Dieses Gemisch wird einer mehrstufigen Kühlung (34) unterworfen und gelangt schließlich zu einem Separator (35), aus welchem man rohes NH₃ durch die Leitung (36) flüssig abzieht. Die gasförmigen Komponenten zieht man in der Leitung (27) ab und führt sie als Kreislaufgas zurück.

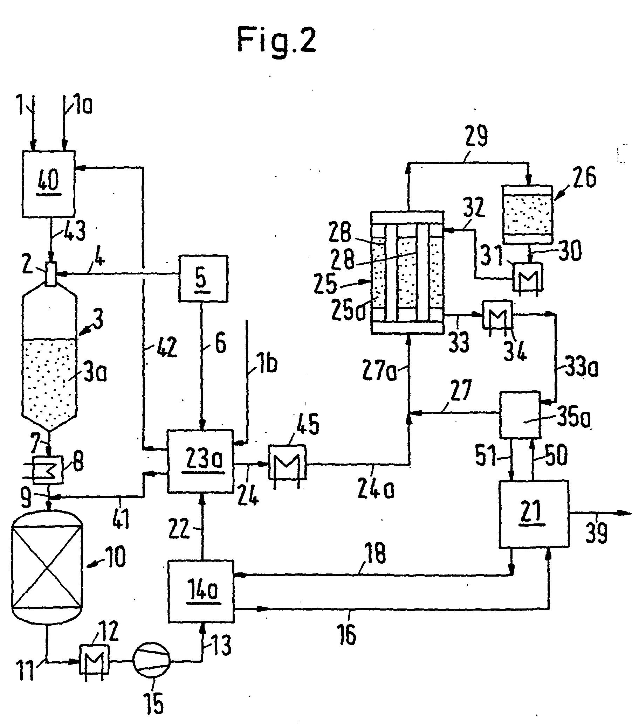

Das erzeugte rohe NH₃ kann ganz oder teilweise durch die Leitung (37) entfernt und einer an sich bekannten Verwendung zugeführt werden. Ferner kann man das rohe NH₃ ganz oder teilweise durch die Leitung (38) einer Harnstoff-Synthese zuführen, die an sich bekannt ist. Erzeugter Harnstoff wird in der Leitung (39) abgezogen. Beim Verfahren der Fig. 2 wird das in der Leitung (11) von der Konvertierung (10) kommende Synthesegas durch die indirekte Kühlung (12) geführt, im Kompressor (15) verdichtet und durch die Leitung (13) einem CO₂-Absorber (14a) aufgegeben. Darin wird CO₂ mit einer schwachen Carbamat-Lösung entfernt, die in der Leitung (18) herangeführt wird und aus der Harnstoff-Synthese (21) kommt. Gebrauchte CO₂haltige Waschlösung wird in der Leitung (16) abgezogen und der Synthese (21) zugeführt. Das teilweise gereinigte Synthesegas strömt in der Leitung (22) zur Feinreinigung (23a), die, z.B. als Flüssig-Stickstoff-Wäsche, als Druckwechsel-Adsorptionsanlage oder als katalytische Methanisierung ausgestaltet sein kann. Die Leitung (1b) ist nur für die Flüssig-Stickstoff-Wäsche sinnvoll.

Die Ammoniak-Synthese arbeitet, wie bereits zusammen mit Fig. 1 beschrieben. Das von der Kühlung (34) kommende Produktgemisch gelangt in der Leitung (33a) in einen Absorber (35a), wo NH₃ mittels Wasser aus der Leitung (50) ausgewaschen wird. Das erzeugte NH₃-haltige Wasser wird in der Leitung (51) der Harnstoff-Synthese (21) zugeführt, Einzelheiten sind in EP-A-0905 127 beschrieben. Im übrigen haben die Bezugsziffern der Fig. 2 die in Fig. 1 erläuterte Bedeutung.

Das Verfahren der Erfindung hat gegenüber bekannten Verfahren insbesondere folgende Vorteile:
1. Auf die Dampfreformierung (steam reforming) wird verzichtet, was den Verzicht auf einen großen und teuren Anlagenteil bedeutet. Gleichzeitig kann man dadurch Vorteilhafterweise beim Cracken von Methan und anderen Kohlenwasserstoffen bei höheren Drücken arbeiten als es in der Dampfreformierung möglich wäre.
2. Der im H₂-N₂-Synthesegas nötige Stickstoff wird vorzugsweise erst in der Wäsche mit flüssigem Stickstoff zugegeben und braucht nicht schon stromauf bei der Erzeugung und Reinigung des Wasserstoffs mitgeschleppt zu werden.
3. In der Wäsche mit flüssigem Stickstoff kann man zweckmäßigerweise auch Methan abtrennen und in den autothermen Reformer zurückführen. Dadurch kann man den Reformer bei möglichst niedrigen Temperaturen von etwa 950 °C betreiben und braucht nicht darauf zu achten, dass das im Reformer erzeugte Gasgemisch methanfrei ist. Man kann ferner einen Erdgasstrom, der mit einem Druck von 10 bis 100 bar herangeführt wird, in der Wäsche mit flüssigem Stickstoff expandieren, um Kälte zu erzeugen (Joule-Thompson-Effekt).
4. In der Wäsche mit flüssigem Stickstoff erzeugt man zweckmäßigerweise auch einen CO-reichen Gasstrom, den man zur CO-Konvertierung zurückführt. Ein Restgehalt an CO im konvertierten Gasgemisch ist somit nicht störend und kann bis zu 8 Vol.-% und zumeist höchstens 4 Vol.-% betragen. Dadurch kann man die robusten und kostengünstigen Eisen-Katalysatoren in der Konvertierung verwenden und kann auf die empfindlicheren Kupfer-Katalysatoren verzichten.
5. Die Gasreinigung mittels Flüssig-Stickstoff-Wäsche ergibt ein hochreines H₂-N₂-Synthesegas, so dass das teilweise Entfernen von Kreislaufgas der NH₃-Synthese ganz oder weitgehend entfallen kann.
6. Die anfallende Abwärme reicht aus, um damit den gesamten Energiebedarf, einschließlich Kompressionsenergie für die NH₃-Synthese und die anschließende Harnstoff-Synthese, zu decken.
7. Der Verbrauch an Erdgas, bezogen auf den unteren Heizwert, liegt bei der NH₃-Herstellung bei nur etwa 27.3 GJ/t und bei der Harnstoff-Herstellung bei nur etwa 19 GJ/t, was gegenüber bekannten Verfahren äußerst niedrig ist. Dieser Erdgas-Verbrauch liegt auch dem folgenden Beispiel zugrunde.
8. Die Anlage zur Durchführung des Verfahrens kann modularisiert werden und sie lässt sich auf einer relativ kleinen Bodenfläche errichten.

### Beispiel:

Man arbeitet mit einer der Fig. 1 der Zeichnung entsprechenden Verfahrensführung, wobei pro Tag 3000 t Ammoniak oder 5263 t Harnstoff hergestellt werden können. Die folgenden Daten sind teilweise berechnet.

Durch die Leitung (1) wird Erdgas und durch die Leitung (1a) Wasserdampf entsprechend einem Molverhältnis Wasserdampf : Kohlenstoff von 2,55 herangeführt. Daten für Mengen, Temperaturen, Drücke und Gaszusammensetzungen (in Vol.-%) ergeben sich aus Tabelle I:

**Tabelle I**

| Bezugsziffer | 1 | 43 | 7 | 11 | 24a | 27a | 33 | 20 |
|---|---|---|---|---|---|---|---|---|
| Menge (t/h) | 92 | 263 | 336 | 357 | 127 | 382 | 382 | 162 |
| Temperatur (°C) | 25 | 65 | 95 | 32 | 168 | 175 | 403 | 32 |
| Druck (bar) | 55 | 61 | 60 | 57 | 137 | 143 | 140 | 3 |

| Zusammensetzung | | | | | | | | |
|---|---|---|---|---|---|---|---|---|
| CH₄ | 91,3 | 27,0 | 1,8 | 2,0 | - | - | - | 0,8 |
| C₂H₆ | 5,8 | - | - | - | - | - | - | - |
| CO | - | 1,6 | 10,6 | 1,1 | - | - | - | - |
| CO₂ | 1,9 | 0,6 | 7,1 | 16,7 | - | - | - | 99,0 |
| Ar | - | - | 0,3 | 0,5 | - | - | - | 0,1 |
| H₂ | - | 3,2 | 38,7 | 47,5 | 74,8 | 70,8 | 54,1 | 0,1 |
| N₂ | 1,0 | 0,3 | 0,4 | 2,3 | 25,2 | 24,4 | 18,9 | - |
| H₂O | - | 67,3 | 41,1 | 29,9 | - | - | - | - |
| NH₃ | - | - | - | - | - | 4,8 | 27,0 | - |

Der Sauerstoff der Leitung (4) hat einen O₂-Gehalt von 95 Vol.-%. Das Synthesegas der Leitung (24) enthält weniger als 5 ppm (vol.) CO und etwa 25 ppm (vol.) Ar. Der NiO-Katalysator (3a) und auch die Katalysatoren der NH₃-Synthese sind handelsüblich (Hersteller z.B. Süd-Chemie, München (DE) Typ G- 90 und AS - 4). Der Reformer (3) wird mit einer Austrittstemperatur von 950 °C betrieben, bei welcher der gesamte Gasverbrauch am niedrigsten ist.

Die Konvertierung (10) arbeitet mit einem ersten, gasgekühlten Reaktor von ähnlicher Bauart wie der Reaktor (25), gefolgt von einem Zwischenkühler und einem adiabatischen Reaktor mit Katalysatorschüttung. Der Konvertierungskatalysator ist ein handelsüblicher Fe-Cr-Katalysator (Typ G - 3C der Süd-Chemie). Der CO-Restgehalt des konvertierten Gases beträgt nur 1,6 Vol.-% (trocken gerechnet), das Volumenverhältnis H₂ : CO₂ ist 2,84 (trocken gerechnet).

Für die Gaswäsche (14, 17) wird das Rectisol-Verfahren verwendet, wobei CO₂ mit Methanol von -58 °C entfernt wird. In der Flüssig-Stickstoff-Wäsche (23) wird das Synthesegas zunächst auf-185 °C gekühlt, wobei CH₄ kondensiert, abgetrennt und durch die Leitung (42) abgezogen wird. Im Kontakt mit flüssigem N₂ wird dann der CO-Gehalt kondensiert, abgetrennt und gelangt in der Leitung (41) zur Konvertierung. Die Zusammensetzung der Ströme in den Leitungen (41) und (42) zeigt Tabelle II (in Vol.-%):

**Tabelle II**

| | (41) | (42) |
|---|---|---|
| CH₄ | 5,13 | 52,54 |
| CO | 21,18 | 12,27 |
| CO₂ | - | 0,53 |
| Ar | 7,18 | 8,64 |
| H₂ | 9,76 | 6,75 |
| N₂ | 56,75 | 19,27 |

Im Kühlsystem (34) werden 65 % des produzierten NH₃ durch Kühlwasser verflüssigt. Das Abziehen eines Teilstromes (purge gas) zum Entfernen von Verunreinigungen aus dem Kreislaufgas der NH₃-Synthese entfällt.

## Patentansprüche

1. Verfahren zum katalytischen Erzeugen von Ammoniak aus einem Stickstoff-Wasserstoff-Gemisch, **dadurch gekennzeichnet, dass** man Erdgas zusammen mit O₂-reichem Gas, welches einen O₂-Gehalt von mindestens 70 Vol.-% aufweist autotherm bei Temperaturen im Bereich von 900 - 1200 °C, einem Druck von 40 bis 100 bar und in Gegenwart eines Spaltkatalysators zu rohem Synthesegas reformiert, welches, trocken gerechnet, einen H₂-Gehalt von 55 bis 75 Vol.-%, einen CO-Gehalt von 15 bis 30 Vol.-%, einen CO₂ - Gehalt von 5 bis 30 Vol.-% und ein Volumenverhältnis H₂ : CO von 1,6 bis 4 aufweist, dass man das rohe Synthesegas aus dem autothermen Reformer abzieht, kühlt, durch eine katalytische Konvertierung zum Umwandeln von CO in H₂ leitet und ein konvertiertes Synthesegas mit einem H₂-Gehalt, trocken gerechnet, von mindestens 55 Vol.-% und einem CO-Gehalt von höchstens 8 Vol.-% abzieht, dass man das konvertierte Synthesegas einer mehrstufigen Gaswäsche zum Entfernen von CO₂, CO und CH₄ unterzieht, wobei man das Synthesegas in mindestens einer Gaswaschstufe mit flüssigem Stickstoff in Kontakt bringt und ein N₂-H₂-Gemisch erzeugt, welches man einer Ammoniak-Synthese zum katalytischen Erzeugen von Ammoniak zuführt.

2. Verfahren nach Anspruch 1, **dadurch gekennzeichnet, dass** man den in der Ammoniak-Synthese erzeugten Ammoniak mindestens teilweise durch Umsetzen mit CO₂ zu Hamstoff umwandelt.

3. Verfahren nach Anspruch 1 oder 2, **dadurch gekennzeichnet, dass** man in mindestens einer Gaswaschstufe CO₂ aus dem konvertierten Synthesegas entfernt, das entfernte CO₂ mindestens teilweise zurückgewinnt und zum Erzeugen von Harnstoff verwendet.

4. Verfahren nach Anspruch 1 oder einem der folgenden, **dadurch gekennzeichnet, daß** man CO₂ aus dem konvertierten Synthesegas in einer physikalischen Wäsche mit Methanol bei Temperaturen im Bereich von -70 bis -20°C entfernt.

5. Verfahren nach Anspruch 1 oder einem der folgenden, **dadurch gekennzeichnet, dass** man in der mit flüssigem Stickstoff arbeitenden Gaswaschstufe CO-haltiges Gas aus dem Synthesegas abtrennt und zur katalytischen Konvertierung leitet.

6. Verfahren nach Anspruch 1 oder einem der folgenden, **dadurch gekennzeichnet, dass** man das N₂-H₂-Gemisch in der Ammoniaksynthese durch mindestens zwei Katalysator enthaltende Reaktoren führt, wobei das N₂-H₂-Gemisch in einem Reaktor als Kühlmedium zum indirekten Kühlen des Katalysators dient.

7. Verfahren nach Anspruch 1 oder einen der folgenden, **dadurch gekennzeichnet, dass** das die Konvertierung verlassende Synthesegas ein Volumenverhältnis H₂ : CO₂ von 2,5 - 3,0 (trocken gerechnet) aufweist.

8. Verfahren nach Anspruch 1 oder einem der folgenden, **dadurch gekennzeichnet, daß** man einen Erdgas-Strom mit einem Druck von 10 bis 100 bar in die mit flüssigem Stickstoff arbeitende Gaswaschstufe leitet und den Erdgas-Strom darin mindestens 8 bar entspannt

## Claims

1. A process for the catalytic production of ammonia from a nitrogen/hydrogen mixture, **characterised in that** natural gas together with O₂-rich gas which has an O₂ content of at least 70 vol. % is reformed autothermally to form crude synthesis gas at temperatures in the range from 900 - 1200°C, a pressure of 40 to 100 bar and in the presence of a reforming catalyst, which gas, calculated in the dry state, has an H₂ content of 55 to 75 vol. %, a CO content of 15 to 30 vol. %, a CO₂ content of 5 to 30 vol. % and a volume ratio H₂ : CO of 1.6 to 4, that the crude synthesis gas is extracted from the autothermal reformer, cooled, passed through a catalytic conversion stage for converting CO into H₂, and a converted synthesis gas having an H₂ content, calculated in the dry state, of at least 55 vol. % and a CO content of at most 8 vol. % is extracted, that the converted synthesis gas is subjected to a multi-stage gas scrubbing process for removing CO₂, CO and CH₄, the synthesis gas being brought into contact with liquid nitrogen in at least one gas scrubbing stage and an N₂/H₂ mixture being produced which is fed to an ammonia synthesis stage for the catalytic production of ammonia.

2. A process according to Claim 1, **characterised in that** the ammonia produced in the ammonia synthesis stage is at least partly converted to urea by reacting with CO₂.

3. A process according to Claim 1 or 2, **characterised in that** in at least one gas scrubbing stage CO₂ is removed from the converted synthesis gas, the CO₂ which is removed is at least partly recovered and is used for the production of urea.

4. A process according to Claim 1 or one of the following claims, **characterised in that** CO₂ is removed from the converted synthesis gas in a physical scrubbing stage with methanol at temperatures in the range from -70 to -20°C.

5. A process according to Claim 1 or one of the following claims, **characterised in that** in the gas scrubbing stage which operates with liquid nitrogen CO-containing gas is separated off from the synthesis gas and is passed on to the catalytic conversion stage.

6. A process according to Claim 1 or one of the following claims, **characterised in that** the N₂/H₂ mixture in the ammonia synthesis stage is passed through at least two catalyst-containing reactors, the N₂/H₂ mixture in one reactor serving as a cooling medium for indirectly cooling the catalyst.

7. A process according to Claim 1 or one of the following claims, **characterised in that** the synthesis gas leaving the conversion stage has a volume ratio H₂ : CO₂ of 2.5 - 3.0 (calculated in the dry state).

8. A process according to Claim 1 or one of the following claims, **characterised in that** a natural gas stream at a pressure of 10 to 100 bar is passed into the gas scrubbing stage which operates with liquid nitrogen and the pressure of the natural gas stream is reduced by at least 8 bar therein.

## Revendications

1. Procédé de production catalytique d'ammoniac à partir d'un mélange d'azote et d'hydrogène, **caractérisé en ce que** l'on reforme du gaz naturel conjointement avec du gaz riche en O₂, qui a une teneur en O₂ d'au moins 70 % en volume, à des températures de l'ordre de 900° à 1 200° C sous une pression de 40 à 100 bar et en présence d'un catalyseur de craquage en du gaz de synthèse brut qui, exprimé en matière sèche, a une teneur en H₂ de 55 à 75 % en volume, une teneur en CO de 15 à 30 % en volume, une teneur en CO₂ de 5 à 30 % en volume et un rapport en volume de H₂ : CO de 1,6 à 4, **en ce que** l'on soutire le gaz brut de synthèse du reformeur autothermique, qu'on le refroidit, qu'on le fait passer dans une conversion catalytique pour la transformation de CO en H₂ et l'on soutire un gaz de synthèse transformé ayant une teneur en H₂, exprimée en matière sèche, d'au moins 55 % en volume et une teneur en CO d'au plus 8 % en volume, **en ce que** l'on soumet le gaz de synthèse transformé à un lavage de gaz à plusieurs étages pour l'élimination de CO₂, CO et CH₄, le gaz de synthèse étant mis dans au moins un étage de lavage de gaz en contact avec de l'azote liquide et il est produit un mélange de N₂ et de H₂ que l'on envoie à une synthèse de l'ammoniac pour la production catalytique d'ammoniac.

2. Procédé suivant la revendication 1, **caractérisé en ce que** l'on transforme l'ammoniac produit dans la synthèse de l'ammoniac au moins en partie par réaction sur du CO₂ en de l'urée.

3. Procédé suivant la revendication 1 ou 2, **caractérisé en ce que** l'on enlève dans au moins un étage de lavage de gaz du CO₂ du gaz de synthèse transformé, on recueille au moins en partie le CO₂ enlevé et on l'utilise pour produire de l'urée.

4. Procédé suivant la revendication 1 ou l'une des suivantes, **caractérisé en ce que** l'on enlève du CO₂, du gaz de synthèse transformé dans un lavage physique par du méthanol à des températures de l'ordre de moins 70 à moins 20° C.

5. Procédé suivant la revendication 1 ou l'une des suivantes, **caractérisé en ce que** l'on sépare dans l'étage de lavage de gaz fonctionnant à l'azote liquide, du gaz contenant du CO ou du gaz de synthèse et on l'envoie à la conversion catalytique.

6. Procédé suivant la revendication 1 ou l'une des suivantes, **caractérisé en ce que** l'on envoie le mélange de N₂ et de H₂ de la synthèse de l'ammoniac dans au moins deux réacteurs contenant du catalyseur, le mélange de N₂ et de H₂ servant dans un réacteur de fluide de refroidissement pour le refroidissement indirect du catalyseur.

7. Procédé suivant la revendication 1 ou l'une des suivantes, **caractérisé en ce que** le gaz de synthèse quittant la conversion a un rapport en volume de H₂ : CO₂, de 2, 5 à 3,0 (exprimé en matière sèche).

8. Procédé suivant la revendication 1 ou l'une des suivantes, **caractérisé en ce que** l'on envoie un courant de gaz naturel sous une pression de 10 à 100 bar dans l'étage de lavage de gaz fonctionnant à l'azote liquide et on y détend le courant de gaz naturel d'au moins 8 bar.
